# EUROPEAN PATENT APPLICATION

(11) **EP 4 327 835 A2**
(43) Date of publication of application: **28.02.2024**
(21) Application number: 22738749.5
(22) Date of filing: 19.04.2022
(51) Int. Cl.: A61L 2/07, A61L 2/20, A61L 11/00, B01J 19/00, B09B 3/00, G09F 3/00, H05B 6/00

(54) **MOBILE STERILIZATION SYSTEM**

(30) Priority: 20.04.2021 MX 2021004545
(71) Applicant: Guirette Hermann Legueu, Jean Louis Pierre Jacques, Houston, TX 77024 (US); Guirette Hentschel, Robert, Houston, TX 77024 (US)
(72) Inventor: Guirette Hermann Legueu, Jean Louis Pierre Jacques, Houston, TX 77024 (US); Guirette Hentschel, Robert, Houston, TX 77024 (US)
(74) Representative: Pons
(86) International application number: PCT/MX2022/050038
(87) International publication number: WO 2022/225385

(57) **Abstract**

Disclosed herein is a mobile sterilization and disinfection system configured to operate under official requirements for the handling of BIHW, adapted to perform the indicated procedures at the place where such wastes or residues are generated, increasing the efficiency of the processes to achieve it and ensuring that the pre-established routes for its transfer do not constitute an additional risk for the persons in charge of the handling. The operation and increased efficiency of the mobile system is a function of temperature diffusion control during the sterilization cycles, as well as joint and system-specific conditions to achieve increased efficiency.

## Description

### INDUSTRIAL PROPERTY RIGHTS RESERVED

A portion of the description of this application contains material subject to copyright protection. The owner of such rights has no objection to the reproduction by facsimile of the patent document or application description by any person, as it appears in the patent file or records in the Patent and Trademark Office, but otherwise reserves all industrial property rights.

### TECHNICAL FIELD

The present application pertains to the field of sterilization, and integral disinfection of both objects and spaces in a manner that facilitates and promotes the mobility of the sterilization system or station.

In the present patent document, a mobile system for sterilization and disinfection (1) of all types of medical waste, air and surfaces is specified.

### BACKGROUND

There are standards for the management of waste and medical waste generated in a hospital, as well as its collection. Examples include "Guidelines for Environmental Infection Control in Health-Care Facilities (2003)", issued by the US CDC, or the Guidelines for the Management of Biological-Infectious Hazardous Waste (BIHW) in Health Units, issued by the Mexican Ministry of Health.

These guidelines point out that, from their generation, handling and during transportation, there are risks for the personnel who handle them, as well as for the hospital patients and personnel who transport them to their final destination. Therefore, such guidelines also indicate specifications on pre-established routes to move the waste safely and quickly from the generating areas to the temporary storage area, avoiding passing through waiting rooms or during patients' meal and/or visiting hours.

It is important to note that there are also specifications related to the feasibility of the medical unit having manual carts for transporting waste, which should not exceed their load capacity to prevent the waste from falling off the carts and being dispersed during their journey.

Considering the guidelines for handling BIHW, the idea arises of a sterilization system configured to perform the sterilization and disinfection procedure at the place where such waste or residues are generated, increasing the efficiency of the processes to achieve this and ensuring that the pre-established routes for their transfer do not constitute a risk for people nearby.

It is important to mention that the traceability of BIHW from its generation to its final destination is fundamental, since several companies with different responsibilities for handling and transportation may be involved in its handling. Under this premise, in a preferential modality, and with the characteristics provided to the BIHW bag, the origin of such waste can be traced at all times.

It is not the intention of this application to modify the standard guidelines for the handling of BIHW, but rather to contribute to make these guidelines safer, more efficient and without increasing costs because it can be used in small medical units avoiding the construction of expensive sterilization facilities such as, for example, buildings.

It is important to point out that, in order to achieve these purposes, high levels of efficiency related to thermal conditioning and sterilization conditions must be achieved under any variable or heterogeneity presented by the waste, where the system that allows it has the means to facilitate its transport to the place where the waste or BIHW is generated.

The device now described includes a set of measures to achieve these levels of sterilization efficiency, since it considers improvements in the components that make up the mobile sterilization system and evaluates the operating conditions according to the characteristics of the load of BIHW in each operating cycle.

Within the improvement of the components of the mobile sterilization system, we can mention the arrangement in the space of the components to increase the efficiency of the thermal conditioning means that control the temperature diffusion in each sterilization cycle by means of joint and particular conditions.

On the other hand, the control of the process conditions is configured to set operating parameters, such as temperature exposure times, steam pressure and volume of the sterilizing agent according to the mass of the BIHW load to be processed.

### DEFINITIONS

BIHW waste. Meets the definitions and classification of BIHW set forth in NOM-087-ECOL-SSA1-2002 and criteria defined by "Guidelines for Environmental Infection Control in Health-Care Facilities (2003)". See "Categories of Medical Waste" of the document consulted in the following electronic address:
https://www.cdc.gov/infectioncontrol/pdf/guidelines/environment al-guidelines-P.pdf

Function control interface (6). It has a screen for interaction between the mobile system and the user. It is configured to determine operation or process parameters, such as pressure, temperature, amount of sterilizing solution and time depending on the type of BIHW load, such as the mass inside the bag. It also has an electronic traceability reader.

Metallic container (3). Made of stainless steel, it has a hermetically sealed lid. Specifications will be addressed in the detailed description of the invention.

Metallic coating of the internal floor of the metallic container (3). Modalities of the invention, consider a gradient surface coating partially reflective of electromagnetic waves. The coating comprises an aluminum alloy grid stamped or printed on an epoxy resin substrate.

Internal floor of the container. In other embodiments, the internal floor of the metallic container (3) comprises a metallic partially electromagnetic wave reflective gradient surface coating (5) or a metallic humped plate (3.3) or combinations thereof.

High performance bag (4). Consisting of HDPE configured to withstand load, pressure and temperature conditions during the sterilization process. Additionally, for the present application, the high-performance bag has an electronic traceability system including, but not limited to QR codes, bar code, SD card, microwave resistant radio frequency identifier (RFID), GPS locator or any other means of traceability that a technician in the field can determine.

Bag for BIHW (8). It can be yellow or red and for this application, it meets the regulatory guidelines of NOM-087-ECOL-SSA1-2002.

Plastic container for sterilizing solution. Polyethylene or biodegradable polymer bag sensitive to temperature increase, such sensitivity considers the rupture of the container for the release of the sterilizing solution in the form of steam. Additionally, for the present application, the BIHW bag (8) has an electronic traceability system that includes, but is not limited to QR codes, bar code, SD card, microwave resistant radio frequency identifier (RFID), GPS locator or any other means of traceability that a technician in the field can determine.

Sterilizing solution. Selected from the group comprising (a) water, (b) aqueous solution of hydrogen peroxide and oxalic acid, and (c) saline solution.

Thermal conditioning means. Selected from the group of electrical resistors and microwave emitting magnetron (2"). They are configured to control temperature diffusion in coordinates.

Conventional means to determine the mass of the BIHW load in the place where they were generated.

### PRIOR ART

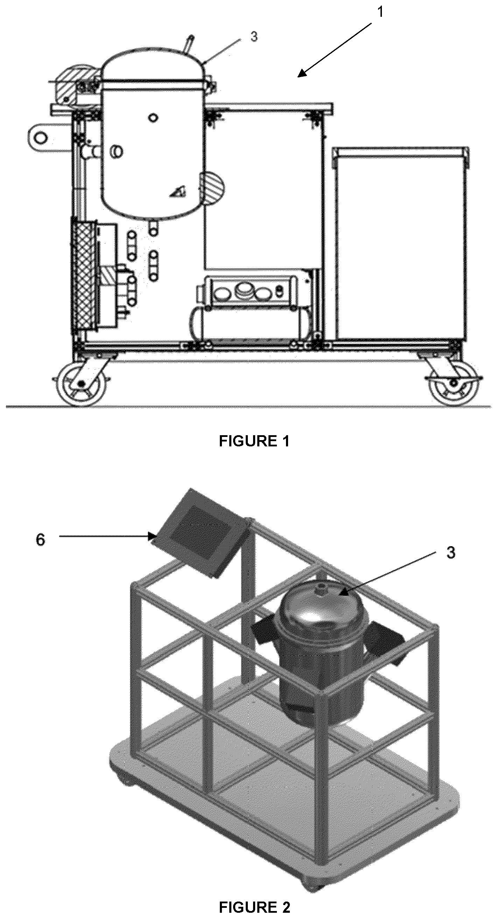

Document US2004112894 describes a sterilizer for surgical and dental instruments. It states that liquid water is rapidly vaporized by microwave heating and steam is generated to achieve a vapor pressure. Micron-size water droplets are intermittently sprayed onto items that are arranged on a tray from both the top and bottom of the tray; this is followed by a plurality of spray/microwave cycles. Figure 1 shows two microwave emitters marked 32 and 48.

It additionally points out that metallic instruments are problematic in microwave-assisted sterilization processes because such instruments reflect microwave energy and, when placed in the microwave field, produce electric arc.

US2010132735 describes a cleaning apparatus for cleaning objects. The cleaning apparatus comprises a cleaning region for cleaning the object using a cleaning liquid. The cleaning apparatus also comprises a microwave disinfecting device having a microwave source for generating microwave radiation.

Additionally pointed out that the cleaning apparatus has at least one temperature sensor for detecting a temperature of the cleaning material. The temperature sensors can also detect, for example, an ambient temperature (e.g., a steam temperature) in at least one cleaning zone and/or in a separate disinfection zone. For example, the sensors used may be infrared sensors and indicates the possibility of being able to employ other types of temperature sensors, e.g., resistive temperature sensors, (thermocouples).

Document KR20060017907 describes a continuous sterilizer apparatus for pathogenic waste using microwaves and steam. It states that, after sterilizing by exposing pathogenic waste to steam having a temperature above atmospheric pressure and boiling point supplied through a steam generator for a predetermined time, the sample is secondarily subjected to a microwave process.

The waste is exposed to steam pressure above atmospheric pressure supplied through the steam tube while being transported on the conveyor belt driven at a predetermined speed by an external motor. The waste discharged through the funnel is sent to the inside of the microwave chamber, where there is a separate conveyor belt to transport the waste. After being sterilized by the microwaves generated by the magnetron.

The document US5223231 describes the sterilization of medical waste or more particularly to the sterilization of medical waste by using a microwave autoclave. The document points out that, the most reliable sterilization method generally recognized is autoclaving in a saturated steam atmosphere for periods of time varying from about ten minutes to a day or more.

US5879643 describes apparatus for heating, disinfecting and sterilizing materials by exposure to microwave radiation; it includes a treatment chamber housing a container filled with material to be treated. An injector is in fluid communication with the container to introduce a liquid into the material. Additionally it points out as part of its process that, the heat contained in the exhaust gas or exhaust vapor is used to raise the temperature of the fresh water supplied thereto and is used for introduction into the container (1) through the conduit and nozzle of the injector.

CA2172653 discloses a method and apparatus for disinfecting or sterilizing infectious waste. Such infectious waste, which must first be disinfected or sterilized before it can receive other treatment, accumulates, for example, in hospitals.

It is further stated that, the infectious waste to be treated remains in the heated saturated steam within the pressure chamber for a predetermined period of time necessary for disinfection or sterilization. The pressure chamber is finally aerated, and the disinfected or sterilized waste can be disposed of.

US6097015 describes a new method and apparatus for sterilizing, disinfecting or heating materials, objects, liquids and the like under pressure. The invention uses the generation and transmission of coaxial microwaves in single mode that do not interfere from multiple sources to the material to be treated.

Document JP2008093231 describes a heat sterilization apparatus for medical waste, which enables heat sterilization at a site where medical waste is generated. The heat sterilization apparatus includes a pressure container for containing medical waste therein, a pressure device for pressurizing the interior of the pressure container, and a microwave oscillator for heating the medical waste with microwaves.

Document JP2004181022 describes a medical waste treatment equipment that improves the property of uniform heating, reduction of processing time, simplification of the main body of the equipment, safety protection of a waste treatment container by microwave control, etc. The equipment is provided with: a pressure container having a lid for opening and closing lia; a container for holding a treatment bag by adding a solvent liquid to a material to be heated in the container; magnetrons 6 and 7 for microwave irradiation; a touch sensor having a detection part for outputting the detection signal of expansion and contraction of the treatment bag; and a controller for controlling the microwave irradiation time with the detection signal of the touch sensor.

On the other hand, it is important to highlight that the present invention is located in the same line of research, development and commercialization as patent application WO2018129107 which, describes a system for sterilizing medical waste includes a pressure tank configured to receive a pressure bag containing the medical waste; a steam generator that introduces steam into the pressure tank through a first pipeline via a connector attached to the pressure tank; and a vacuum compressor that removes fluids from the pressure tank. The pressure tank, the steam generator and the vacuum compressor are connected so that the fluids within the pressure tank are contained. The system described in said application features: pipes, components or mechanisms that capture condensation and reintroduce it into a new batch of medical waste to be treated; altogether, the arrangement of said system, as shown in their respective figures, prevents in situ sterilization and thus requires, among other actions, transportation of the waste to the location of said system. The present system eliminates, in addition to stages of the method, within its arrangement, costly specialized installations for the equipment, as well as piping and external components for the generation and handling of steam before and after contact with the biohazardous and infectious waste.

As can be appreciated, the state of the art is wide regarding sterilization devices, however, within the search for process optimization, the inventors have developed a mobile system for sterilization and disinfection of medical waste, air and surfaces with novel and inventive variables that make the process more efficient, satisfying local and international regulations for the handling and transport of BIHW.

### SUMMARY

As described above, the device or system now presented considers variables such as the mass of the generated BIHW to determine the operating cycles through a function control interface (6) for interaction between the system and the user, configured to determine thermal conditioning conditions, steam pressure, time, and sterilizing solution supplies to generate steam inside the BIHW bag (8); taking into account the particular characteristics of the contents of the load that is subjected to the sterilization and disinfection treatment.

To carry out the calculation of cycles and operating conditions, the mobile system has the means to determine the mass of the BIHW load at the place where they were generated. Once the variable data is entered, the system evaluates, by means of the function control interface (6), whether the BIHW bag (8) meets the criteria of NOM-087-ECOL- SSA1-2002, where a load greater than 80% of the volume capacity of the bag prevents the start of the disinfection and sterilization process. The control interface (6) also determines and ensures the presence of the plastic container for sterilizing solution. The traceability of both components is achieved by means of an electronic traceability reader. A load of more than 80% of the capacity of the BIHW bag (8) and/or the absence of the plastic container for sterilizing solution prevents the start of the disinfection and sterilization process, i.e. the thermal conditioning means are instructed not to start operations.

Under the premise of respecting local and international regulations in the handling of BIHW, the mobile sterilization and disinfection system establishes operation or process parameters, such as pressure, temperature, amount of sterilizing solution and time depending on the type of BIHW load, as well as the mass of the load inside the BIHW bag.

At this point, the inventors of the present mobile sterilization system found that each variable is important to increase the efficiency of the equipment, therefore, the thermal conditioning means are also treated as part of the system or set of components. In this regard, it is important to note that, the metal container (3) comprises a plurality of radio frequency diverters configured to control temperature diffusion in coordinates.

In other embodiments, the inner floor of the metal container (3) has a surface coating (5) of gradient partially reflecting electromagnetic waves to achieve sterilization conditions with different characteristics. To achieve different temperature diffusion characteristics, a grid of aluminum alloy strips (7) stamped on an epoxy substrate is used to modify the temperature diffusion by means of said grid on the lower floor of said container. In other embodiments, the inner floor of the metal container comprises a surface with metal bumpers or humps (3.3). In other embodiments, the container comprises a combination of the above: the partially reflective gradient surface coating (5) and the bumped surface (3.3), as described below.

Concerning the surface coating (5) of partially reflective gradient of electromagnetic waves, it is important to mention, that they are developed by means of a grid of aluminum alloy strips (7) stamped on an epoxy substrate. In this regard, please find in one embodiment, the grid with strips stamped in an alternating arrangement oriented to the electric field and another section of strips oriented to the magnetic field on the inner floor of the metal container (3), sufficient to achieve a different or modified diffusion control of temperature.

In preferred embodiments, the thermal conditioning means are one or more microwave emitting magnetrons, or one or more electrical resistors.

In one preferred embodiment, it is noted that the thermal conditioning means are microwave emission magnetrons. In this regard, it is important to mention that, the process of applying microwaves to metallic objects is usually not recommended, especially when the BIHW, according to local and international regulations, includes all types of waste, including metallic objects. Therefore, sterilization of such objects by microwave thermal conditioning should be achieved by preventing the electric arc.

In accordance with the definitions of local and international regulations, a normal or typical load of BIHW may contain: "sharps; non-anatomical waste such as gauze, swabs or saturated fields, soaked or dripping body fluids and secretions of patients with tuberculosis or hemorrhagic fevers; pathological waste; liquid blood and its derivatives, as well as disposable utensils used to contain, transfer, inoculate and mix cultures of biological-infectious agents and biological samples for analysis, or combinations thereof, therefore, each load of BI HW to be processed can be as heterogeneous or different from the previous one as from the subsequent one.

In view of this diversity of load types or load mixtures of BIHW to be sterilized and disinfected, the inventors have found that the particular processing conditions are most efficient if precisely matched to the specifications of the load in each cycle. For this reason, the mass determination of the BIHW load prior to each cycle is an important factor in making the sterilization process more efficient.

Likewise, as previously indicated, the efficiency of the process is achieved through a set of variables to be satisfied, where these variables are a function of the radio frequency distribution inside the metal container, particularly in each sterilization cycle. Under these estimations, it is important to point out that the volume of the sterilizing solution is calculated to provide the necessary amount of steam regardless of the type of BIHW load, which should never exceed 80% of the BIHW bag load capacity according to the regulations.

It is worth mentioning that prior to the development of the mobile disinfection system pertaining to this application, the inventors faced several obstacles, including:
The use of a bi-directional flow of steam (in and out of the bag) with the problem of creating a potential for backflow of contaminated gases into the sterilizer. The technically versed can anticipate, in these cases, the existence of a complex network of piping and pumps for fluids entering and exiting the system where the sterilization process takes place. The present system utilizes the efficient sterilization properties of steam without resorting to additional non-localized steam injection and recirculation. This allows the steam to be contained and generated entirely where the BIHW are located, thus eliminating the possibility of cross-contamination of the system or personnel.

Other devices do not meet the requirements for BIHW bags to be considered "hermetically sealed," while the current closure method and heating method meet that standard in two ways: the hermeticity of the BIHW bag and the hermeticity of the metal container.

Other devices require additional components such as steam injectors and recirculation piping, which makes them heavier, while the current thermal conditioning system is comparatively simpler and lighter.

Typically, steam sterilization requires a boiler which makes the system bulky, heavy and difficult to maneuver. The present system uses, in preferred embodiments, radio frequency waves in the microwave spectrum to heat the water or sterilizing solution contained within the high-performance bag and convert it to steam. The steam produced then sterilizes the contents of the contents of the high-performance bag by transferring that heat from the steam to the contents. Additional steam injection according to the state of the art is also eliminated.

### DESCRIPTION OF THE FIGURES

Figure 1. Schematic of the mobile sterilization system.
Figure 2. 3-D isometric projection of the mobile sterilization system.
Figure 3. Hermetically sealed metal container (3), diagram of high-performance bag (4) and arrangement in space of thermal conditioning means including radio frequency diverters.
Figure 4. Diagram of the bottom of the metal container with angular indication of inclination for three radio frequency diverters.
Figure 5. Hermetically sealed metal container (3), diagram of high-performance bag (4) and arrangement in space of thermal conditioning means including a radio frequency diverter with convex surface.
Figure 6. Diagram with isometric view of the bottom wall or internal floor of the container comprising metal stops or humps (3.3).
Figure 7. Isometric projection and close-up view of aluminum alloy strip grid (7).
Figure 8. Two-dimensional graph related to the temperature control volume according to the radial variable.
Figure 9. High-performance bag clamping strap.
Figure 10. Cinch strap in "closed" position.
Figure 11. High-performance bag (4) with holes for fastening strap entry.
Figure 12. High-performance bag (4) with fold and fastening strap in "open" position.
Figure 13. High-performance bag (4) with fold and fastening strap in "closed" position.
Figure 14. Comparative graph of temperature diffusion control; microwave performance.

### DETAILED DESCRIPTION OF THE INVENTION

The mobile sterilization system (1) now described, as indicated, includes a set of means for achieving high levels of efficiency in the sterilization process, since it considers particular improvements of its constituent components and is configured to evaluate the operating conditions according to the particular characteristics of the BIHW load in each operating cycle.

The basic version of the mobile sterilization system (1) comprises thermal conditioning means (2), wherein preferred modalities consider the inclusion of at least one electrical resistance (2') or at least one microwave emitter (magnetron) (2") directed to the interior of the hermetically sealed metal container (3) to control the temperature diffusion efficiently in order to generate the vapor pressure conditions necessary during the sterilization process. When the thermal conditioning means are microwave emitters, the typical operating frequency is at 2450 MHz. An additional benefit of using microwaves within the sterilizer is that they provide an alternate and separate mechanism for dry sterilization prior to the steam generation of the sterilizing solution in addition to providing the thermal conditions within the container.

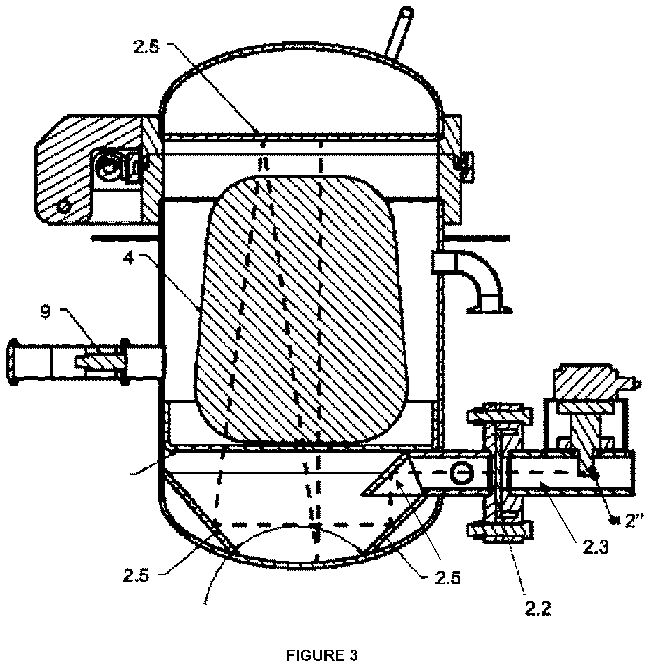

Under the estimation that the thermal conditioning means (2) are microwave emitters, one has the presence of at least one magnetron (2"), a magnetron power supply plate (2.1), a magnetron mounting waveguide (2.3) with locking flange (2. 2), a magnetron waveguide pressure window, a radio frequency choke coil, (also known as RF choke, not shown in Figure 3), a magnetron waveguide container inlet (2.4) and a plurality of microwave waveguide diverters (2.5).

If the characteristics of the BIHW load vary, the conditions are adjusted to the type of materials to be processed. To achieve maximum steam penetration, the nature of the BIHW is determined and a particular configuration is defined. The container (3) is configured to have an adjustable internal pressure, the active control of which is manually / or automatically / or passively regulated. This is done to vary the vapor pressure conditions inside the hermetically sealed high-performance bag (4).

Among the improvements to the components of the mobile sterilization system, mention can be made of the arrangement, inclination and orientation of the radio frequency diverters which, as a whole, increase the efficiency of the thermal conditioning means with the objective of controlling the temperature diffusion in coordinates to the interior of the metallic container (3) in each sterilization cycle.

**Table 1. Saturated steam pressure at different temperatures and suggested time in autoclave**

| **ABSOLUTE PRESSURE** | **TEMPERATURE** | **TIME** |
|---|---|---|
| PSI / Kg/cm² | °C | Minutes |
| 00.00 / 0.0 | 100 | - |
| 5.83 / 0.4 | 109 | - |
| 14.22 / 1.0 | 119.6 | - |
| 15.64 / 1.1 | 121 | 15 - 30 |
| 21.33 / 1.5 | 126.8 | 12 - 15 |
| 25.74 1.8 | 131 | 11 - 13 |
| 28.45 / 2.0 | 132.9 | 10 - 11 |
| 31.3 / 2.2 | 135 | 7-10 |
| 42.67 / 3.0 | 142.9 | - |
| 49.78 / 3.5 | 147.2 | - |

Table 1 shows saturated steam pressure values at different temperatures and the suggested time for autoclave sterilization; the times not established in Table 1 are not included because conditions are suggested to ensure total sterilization of the load in each process. Local standards indicate the optimal conditions to achieve effective sterilization: a high-performance bag containing an BIHW bag is placed, where the high performance bag (4) is resistant to moist heat under sealed conditions in the autoclave with the following conditions: 121° C at 15 PSI (~ 1. 1 Kg/cm2) pressure for at least 30 minutes, (in some cases, up to 90 minutes is suggested) indicating that, under this circumstance, disposable petri dishes and other plastic devices used in the laboratory become "unrecognizable" according to the definition of the "loss of the physical and biological-infectious characteristics of the object so as not to be reused" or after treatment "by steam sterilization, where the waste can be safely handled and disposed of with all other non-hazardous solid wastes in accordance with state solid waste disposal regulations" within the emission standards, in accordance with the previously stated regulations.

For the operation of the present mobile sterilization system, it has been determined that each variable is important to increase the efficiency of the equipment, therefore, the thermal conditioning means (2) are an essential part of the system. Thus, the thermal conditioning means comprise a plurality of radio frequency diverters configured to control the temperature diffusion in coordinates. Figures 3, 4 and 5 show that said plurality of diverters comprise extended surfaces disposed on an inner surface of the metal container, the extended surfaces being in contact with microwaves, and in turn, are oriented to form a temperature gradient inside the metal container (3). The extended surfaces include highly microwave reflective portions, a defined orientation and angular inclinations for temperature diffusion control.

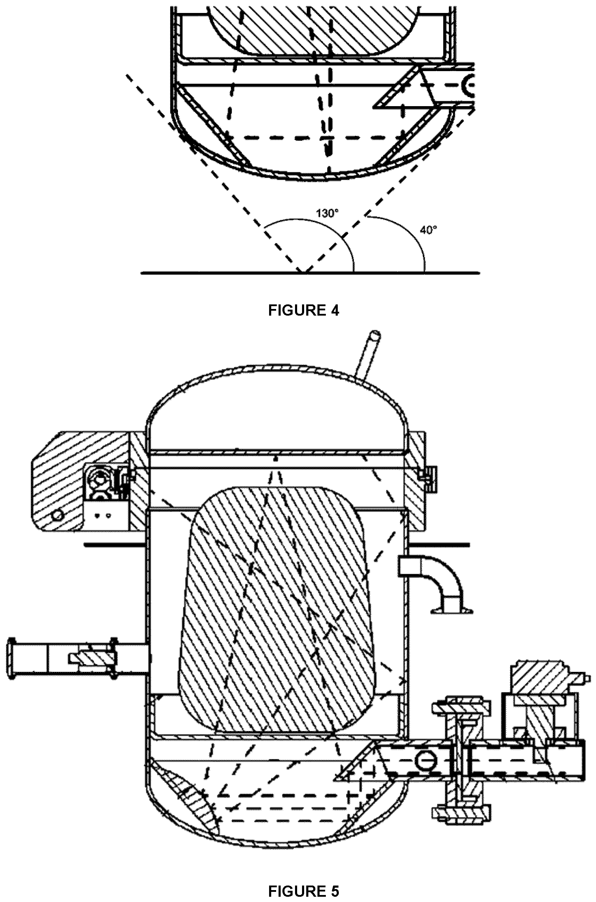

In preferred embodiments, at least two of the extended surfaces comprise highly microwave reflective portions with parallel orientation and both with an angular inclination within the range of 25 degrees to 55 degrees (25°-55°). See Figure 4.

In preferred embodiments, at least one of the extended surfaces comprises highly microwave reflective portions with angular inclination greater than 90°, preferably within a range of 125° to 150° and, at least one other of the extended surfaces comprises highly microwave reflective portions with a horizontal arrangement. See Figures 3 and 4.

Under the above immediate modalities, the extended surfaces constituting the plurality of diverters are planar surfaces. Additionally, the magnetron waveguide (2.3) is arranged at the entrance of the container and in contact with a first extended surface comprising a highly microwave reflective portion facing downwardly and wherein said extended surface has an angular inclination preferably of between 40° to 45°.

In other modalities, at least one extended surface constituting to the plurality of diverters is a convex surface. See figure 5.

In other modalities, the inner floor of the metallic container (3) has an electromagnetic wave partially reflective gradient surface coating (5) to achieve a modified coordinate temperature diffusion. To achieve this, a grid of aluminum alloy strips (7) patterned on an epoxy resin substrate is used on the inner floor of the metal container (3).

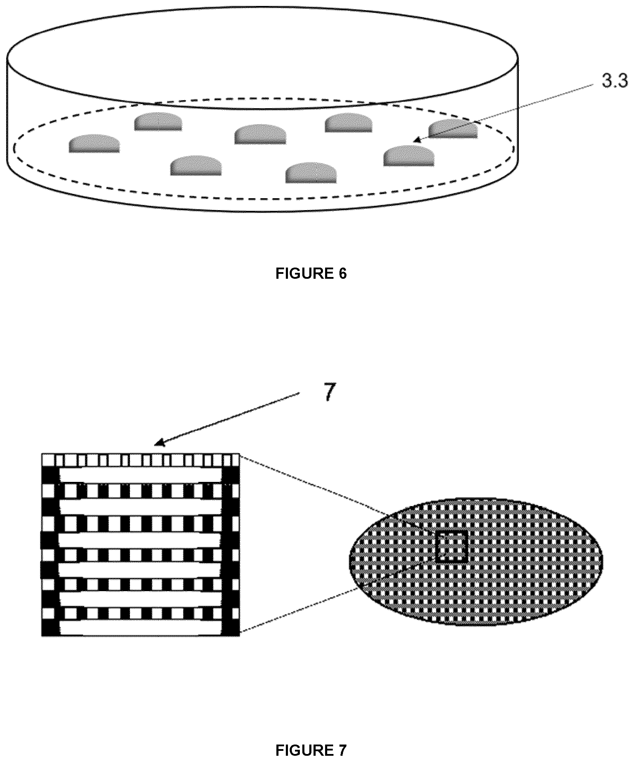

In said embodiment, the electromagnetic wave partially reflective gradient surface coating (5) is defined by a grating (7) composed with a first set of aluminum alloy strips in a perpendicularly oriented arrangement with respect to a second set of aluminum alloy strips, both sets being patterned on an epoxy resin substrate (crystal resin) on the inner floor of the metallic container (3). The coating favors a modified temperature diffusion in coordinates with respect to the diffusion obtained when the plurality of diverters are only extended surfaces. The grating (7) is printed on an epoxy resin substrate with a thickness of between 0.0mm and 0.4mm. The thickness of the aluminum strips is between 0.5mm and 0.4mm, preferably 0.2mm. The width of the strips is about 1 cm and they are placed alternately with a spacing of about Icm to form a set of strips, (see figure 7). The inventors have found that, by this set of adaptations to the system, once the thermal conditioning means (2) begin to operate, a modified temperature diffusion in coordinates to the interior of the metal container (3) is achieved while achieving the desired sterilization conditions.

In other embodiments, the interior floor of the metallic container (3) comprises a surface with metallic bumpers or humps (3.3) partially reflecting electromagnetic waves to achieve a modified temperature diffusion in coordinates. See figure 6.

Within the same objective of increasing efficiency by means of controlled temperature diffusion to the interior of metallic container (3) and under the estimation that the thermal conditioning means (2) are microwave emitters, the inventors have found the following considerations:
The microwave emission source, i.e. the at least one magnetron (2") is located outside the metal container (3), these two components, the metal container (3) and the at least one magnetron (2") being joined by a magnetron mounting waveguide (2.3) with locking flange (2. 2), wherein the guide contains a microwave guide pressure window (2.3), wherein the window material is unfilled extruded polyetherimide (PEI) (Mitsubishi Duratron^{®} U1000 PEI plate), a radio frequency choke coil, ((2.4) also known as RF choke) and with the microwave guide diverter (2.5) with an input angle of angular tilt within a range of 25 degrees to 55 degrees (25°-55°). Under this configuration arrangement of the plurality of diverters, it is possible to control the temperature diffusion in coordinates by generation of temperature gradients inside the metal container (3). See figures 3 and 4.

It is not the purpose of this application to explain the volumetric energy distribution balances for a microwave sterilization process. On the contrary, it is the objective of this application, as a technical improvement, to provide and describe, tangible physical means to control temperature diffusion in volumetric coordinates by generating temperature gradients inside the metal container (3) during the process of steam sterilization of medical waste or BIHW.

The arrangement described above provides a particular interaction between the plastic container for the sterilizing solution, the sterilizing solution and the microwave radiation, which favors and makes the sterilization process more efficient, as will be seen below. The mobile sterilization system is configured to carry out the calculation of cycles and operating conditions; For this, it has means to determine the mass of the BIHW load in the place where they were generated and once the data are entered into the control interface (6), the system is configured to evaluate whether the BIHW bag (8) meets local and international criteria, that is, the existence of a load greater than 80% of the capacity of the volume of the bag, prevents the start of disinfection and sterilization process, that is, the thermal conditioning means are ordered by the interface (6) not to start operations.

The control interface (6) also determines and ensures the presence of the plastic container for sterilizing solution by reading the traceability code; its absence inside the metal container (3) prevents the start of the disinfection and sterilization process; i.e., in this case, the thermal conditioning means also receive the order not to start operations. In this sense, the mobile sterilization system, by means of the function control interface (6), sets operation or process parameters, such as steam pressure, temperature and time depending on the type of BIHW load and the mass inside the BIHW bag. The amount of sterilizing solution is fixed in each of its containers.

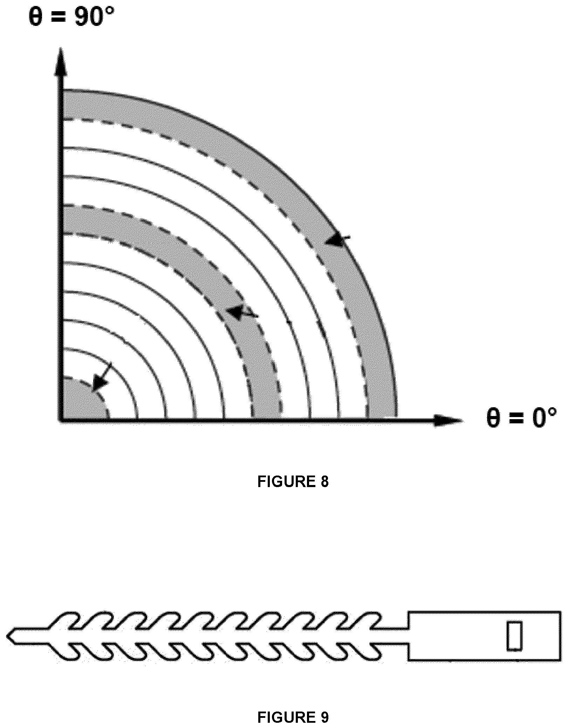

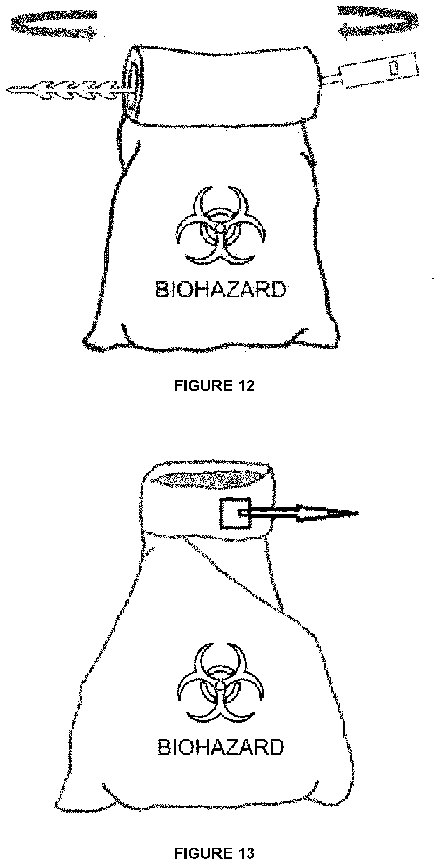

At this point it is important to note that the high-performance bag (4) and the BIHW bag (8) are different. Figures 9, 10 and 11 show, respectively, a fastening strap for the high-performance bag (4) and the same fastening strap in the "closed" position, as well as a high performance bag (4) with holes for the fastening strap entry. For a hermetic seal, once the band is inserted through the holes, the bag is folded downwards, as shown in figure 12, and then the fastening band is placed in the "closed" position, as shown in figure 13. This action ensures a hermetic seal of the high-performance bag (4); the sterilization gases generated inside the bag during the process do not escape from the bag, thus avoiding the creation of a potential backflow of contaminated gases into the metal container (3).

Under the provisions described above, the system provides a particular interaction between the plastic container for sterilizing solution, which is located inside the BIHW bag (8) and the microwave radiation, which favors and makes the sterilization process more efficient.

It is important to note that each load of BIHW is different in each sterilization cycle, due to the heterogeneity of waste that can be produced in a medical unit. Therefore, in order to make the sterilization process more efficient, it is essential to control the temperature diffusion in coordinates.

According to previous disclosure models, it is known that radio frequency or electromagnetic waves satisfy energy conservation for nodes from which the temperature at discrete points can be determined. In practice, one has that, small regions or specific volumes are bound to such nodes with temperature variation as a function of the radial variable. See shaded regions in Figure 8, which represent volumetric regions with different temperatures.

According to this description, the incidental waves have a nodal behavior, so the model and the measurements indicate that spherical regions of the same object have different temperatures; using the model of figure 8, it can be exemplified that the shaded regions are at a higher temperature than those volumetric regions that are not shaded.

During the sterilization process, once the sterilizing solution container breaks due to its sensitivity to temperature increase, steam generation begins to reach sterilization conditions; when the steam is below the critical temperature to remain steam, it condenses and begins to absorb more microwave energy, which causes the water to heat up and vaporize again into functional sterilizing steam. The rate at which steam is heated by microwaves is substantially less than that of water. In practice, this means that a cycle is created within the high-performance bag (4) because liquid water absorbs a higher percentage of the total microwave energy delivered into the system than water vapor. The water vapor penetrates more efficiently and transfers the energy to the contents of the high-performance bag (4) which distributes the vapor and liquid throughout the BIHW load. For this reason, new steam injection pipes are eliminated. The described phenomenon allows a fixed amount of sterilizing solution per sterilization cycle.

Since the system is completely self-contained within the high-performance bag, no injection of fresh steam into the system is necessary to overcome the lower temperature condensed steam that has transferred its energy to the system and its contents to reach and maintain the required temperature and pressure.

The aim of the inventors is, therefore, that the temperature diffusion is controlled at defined coordinates. That is, to achieve a control of the temperature diffusion to ensure an efficient interaction between the contents of the BIHW bag and the radio frequency waves from the thermal conditioning means; such control results in the formation of temperature gradients, so that a defined control volume can be determined, considering the nature of the energy distribution due to microwaves in the sphere model (see shaded areas of Figure 8) and the position in space where the high performance bag (4) is placed inside the hermetically sealed metal container (3). In this sense, control is pursued through tangible physical means, so that the incidental radio frequency waves (microwaves) to the interior of the bag for BIHW are directed to discrete points and the temperature generated by steam inside is also controlled, regardless of the type of BIHW in question. That is, it does not matter if it is a load with metallic waste such as scalpels or soaked or dry gauze or mixtures thereof; the present sterilization system operates independently of the particularity of each load of BIHW.

The microwave energy propagates and refracts within the internal volume of the hermetically sealed metal container (3). The distribution and intensity of the microwaves within the container are a function of the internal geometry of the system, as well as the contents inside the BIHW bag. Based on controlled tests, the inventors have observed that by installing a plurality of diverters inside the container, whose shape and position favor that the reflected microwaves can be controlled to increase the microwave energy interacting with the sterilizing solution container, its subsequent rupture and generation of steam from the sterilizing solution. The configuration that yielded the best control results through various tests was as follows:
Microwaves are produced at the radio frequency source (magnetron) and directed to the hermetically sealed metal container (3) through a waveguide (2.3). The waveguide is connected to the metal container with a sealing flange connection (2.2) featuring an integrated RF choke coil and a pressure sealing window. This locking flange connection does not require the use of conductive materials as is common in the industry and is rated for significantly higher pressure than traditional radio frequency systems can operate. The waves are then deflected into the waveguide before entering the interior of the metal container. Once inside the metallic container, the waves are reflected by a plurality of radio frequency diverters (2.5) to govern the wave distribution and form a temperature gradient. As disclosed above, one end of the magnetron waveguide (2. 3) is arranged at the entrance of the container and in contact with a first extended surface comprising a highly microwave reflecting portion facing downward and wherein said extended surface has a defined angular inclination; the first wave distribution inside the metallic container is not directed towards the high-performance bag (4); with this, the inventors have achieved that the temperature diffusion is not random; the plurality of diverters, the inclination and orientation of diverters reflect the microwaves then towards the top of the container before being redirected towards the load of BIHW contained in the high performance bag from above. See dotted lines in Figures 3, 4 and 5.

The function of the thermal conditioning means (2) is to increase the temperature inside the hermetically sealed metal container, which contains a high-performance bag (4), which in turn contains an BIHW bag (8) inside which there is a plastic container with sterilizing solution and the BIHW. The plastic container for sterilizing solution is selected from the group of polyethylene or biodegradable polymer sensitive to temperature increase, that is, it must break by thermal degradation, release the sterilizing solution to generate sterilization steam.

In this regard, the inventors have determined that to increase the sterilization efficiency of BIHW, the sterilizing solution is selected from the group comprising a) water, b) aqueous solution of hydrogen peroxide with oxalic acid and c) saline solution. In preferred embodiments, the sterilizing solution satisfying (b) above contains from 5 to 7% hydrogen peroxide and oxalic acid in the range of 4 to 6%, the remainder being water. As part of the set of measures to improve efficiency in the sterilization process, the function control interface (6) is configured to detect the plastic container for sterilizing solution, since, without this component, the system does not start operations. With this measure, in addition to the traceability of the plastic container for sterilizing solution, the operator is prevented from starting the process without the means to generate steam to carry out the process.

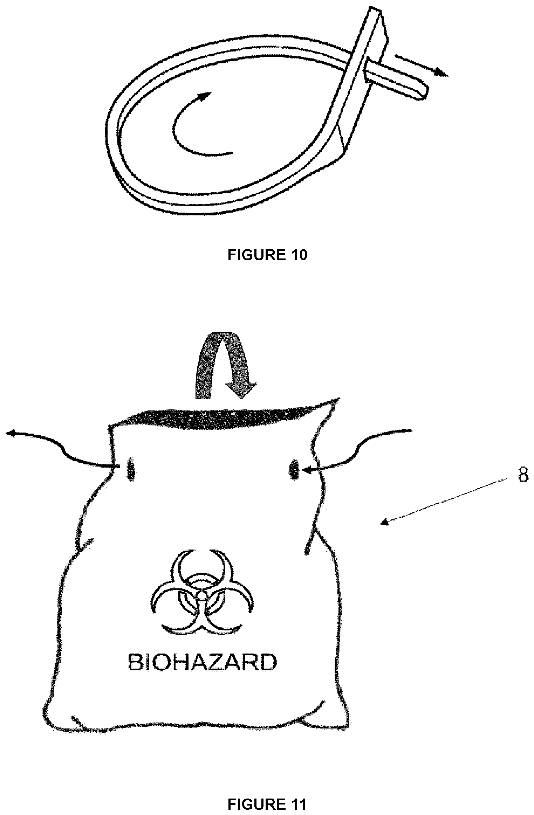

Given the above concepts, part of the general actions to be taken are as follows:
Determine that the mass of BIHW inside the BIHW bag satisfies handling criteria and regulations;
The BIHW bag (8) is placed inside the high-performance bag (4) and hermetically sealed by means of a band. See Figures 11, 12 and 13;
The high-performance bag (4) is placed (suspended/supported) inside the hermetically sealed metal container;
The steam pressure within the system is adjusted to the desired values of pressure, temperature and time to obtain optimum sterilization characteristics.
The thermal conditioning means are turned on and the system can operate for the predetermined cycle time.

In this regard, the thermal conditioning media need not run for the entire cycle once the thermal properties of the BIHW are known and a compliant temperature curve is developed.

Once the BIHW has been maintained at the required temperature for the required cycle time, the system enters a cooling cycle that reduces the temperature inside the container and allows any gas to condense inside the high-performance bag, containing the emission of unpleasant odors, since, once the high-performance bag is sealed, it does not re-open. The pressure release of the metallic container is free of contact of sterilization gases with the environment, where another of the objectives and technical advantages of this sterilization system is the containment of odors emitted by the BIHW. The arrangement of the system's elements prevents odors from escaping from the equipment, which results in a technical advantage that facilitates the transportation of the equipment within the medical units. The above, considering that an impediment to the use of this type of system is the unpleasant odors generated by the decomposition of organic matter in the BIHW.

Once the high-performance bag (4) has reached sterilization conditions by means of pressure, temperature and predetermined time, the high-performance bag can be removed and the system can be restarted with a new load of BIHW.

The mass of BIHW is obtained before the sample is placed in the container. This variable is used to set the process time, defined as the time from when the temperature of the material in the high-performance bag reaches ambient temperature until the internal temperature reaches the minimum sterilization temperature. Different sample masses or loads result in different process times to ensure that sterilization conditions are met, however, time and pressure conditions are maintained in accordance with local and international standards.

The specifications and components for performing each of these actions are framed to the characteristics outlined in this application, so the following aspects of the mobile system are also included:

### - Pressure control

Hermetically sealed metal container, b. Air pressure regulator, c. Air inlet solenoid, d. Inlet air check valve, e. Air compressor, f. Pressure release valve and g. Exhaust air solenoid.

### - Microwave

Magnetron, b. Magnetron power supply plate, c. Magnetron mounting waveguide with locking flange, d. Radio frequency choke coil, (also known as RF choke) , e. Magnetron waveguide pressure seal window, f. Inlet to container from magnetron waveguide and g. First magnetron waveguide diverter.

### - Cooling

Magnetron cooling fan, b. Magnetron power supply plate cooling fan, c. Control board cooling fan, d. Sample cooling circulation fan, e. Sample cooler heat exchanger, and f. Sample cooler cooling fan. Heat exchanger cooling fan.

### - Filtration

Trolley cooling inlet filter, b. Trolley cooling UV sterilizer, c. Trolley cooling fan and d. Trolley cooling outlet duct.

### - Control

Control board, b. Interface display, c. Relay board, d. USB sample scale, e. Metal container air pressure sensor, f. Non-contact IR temperature sensors,
b. Heat exchanger inlet temperature sensor, and h. Heat exchanger outlet temperature sensor.

### 6. - Power.

a. 120v / 240v AC power cord and supply box, 120v / 240v AC to 12v DC charger, c. 12v DC deep cycle high-capacity battery, and d. DC bus control system.

### 7. Auxiliary

a. UV sterilizer LED on the bottom of the mobile structure.
   Having stated the above, the mobile sterilization and disinfection system now disclosed comprises: means for determining the mass of the BIHW load at the place where they were generated; a function control interface for interaction between the mobile system and the user; a hermetically sealed metal container comprising: a high-performance bag inside which there is a bag for BIHW with a maximum load of 80% or less, a plastic container with sterilizing solution for steam generation and the BIHW; pressure control means; thermal conditioning means;
   wherein:
      the thermal conditioning means comprise: a plurality of radio frequency diverters configured to control temperature diffusion in coordinates.

In preferred embodiments, the plurality of diverters are formed by extended surfaces disposed on at least one interior surface of the metallic container, the extended surfaces being in contact with microwaves, and said extended surfaces are oriented to form a temperature gradient inside the metallic container.

In other embodiments, the extended surfaces include highly microwave reflective portions, defined orientation and angular inclinations for temperature diffusion control; the following variants are cited:
at least two of the extended surfaces comprise highly microwave reflective portions with parallel orientation and both with angular inclination within a range of 25° to 55°, wherein said highly reflective portions face each other;
at least one of the extended surfaces comprises highly microwave reflective portions with angular inclination greater than 90°, preferably within a range of 125° to 150°;
at least one of the extended surfaces comprises highly microwave reflective portions facing downward with a horizontal arrangement at the top of the metal container; one end of the magnetron waveguide (2.3) is disposed at the entrance of the container and in contact with a first extended surface comprising a highly microwave reflective portion facing downward and wherein said extended surface has an angular inclination of between 25° to 55°, preferably of between 35° to 45°, and more preferably of between 40° to 45°.

In other modalities, the system additionally comprises thermal conditioning means which may be microwave emitters, or electrical resistors, and additionally comprises cooling means and filtration means.

In other modalities, the partially electromagnetic wave reflective gradient surface coating on the inner floor of the metallic container is defined by a grid composed with a first set of aluminum alloy strips in an arrangement oriented perpendicularly with respect to a second set of aluminum alloy strips, both sets being patterned on an epoxy resin substrate.

In other embodiments, the aluminum alloy forming the grating is printed on an epoxy resin substrate with a thickness of between 0.05mm and 0.4mm. The thickness of the aluminum strips has in turn, a thickness of between 0.5mm and 0.4mm, preferably 0.2mm, and wherein the aluminum alloy comprises Cu 0.03- 0.25%; Fe 0.2-0.5%; Mn 0. 5-2.0%; Si 0.05-0.5%, Zn 0.01-0.095% and unavoidable impurities 0.01-0.1% and Al balance, preferably Cu 0.15%; Fe 0.4%; Mn 1.0%; Si 0.25%, Zn 0.05% and unavoidable impurities 0.05% and Al balance.

Likewise, when the thermal conditioning means are at least one microwave emitter magnetron (2"), the latter is located outside the metal container (3), these two components, the metal container (3) and the at least one magnetron (2") being joined by a magnetron mounting waveguide with locking flange (2. 2), wherein the magnetron mounting waveguide with locking flange (2.2) contains a microwave guide pressure window (2.3), wherein the window material is unfilled extruded polyetherimide (PEI) and the first microwave guide diverter (2.5) has an angle of between 40° to 45°.

In another preferred embodiment, the sterilizing solution is selected from the group comprising a) water, and b) aqueous solution of hydrogen peroxide and oxalic acid, wherein embodiment b) is preferably an aqueous solution containing 5 to 7% hydrogen peroxide, 4 to 6% oxalic acid and the remainder water.

In other embodiments, the mobile sterilization system additionally comprises UV emitters for floor disinfection and the high-performance bags have an electronic traceability system including but not limited to QR codes, barcode, SD card, microwave resistant radio frequency identifier (RFID), GPS locator or any other means of traceability that a person skilled in the art can determine.

In this regard, the present application also describes a method of sterilizing and disinfecting in accordance with the mobile system specification described above,
wherein the method comprises the steps of:
determine the mass of the BIHW load at the site where the waste was generated;
calculate, by means of a control interface, the sterilization and disinfection conditions according to the requirements of step i);
to introduce a load of BIHW and a plastic container with sterilizing solution in a BIHW bag at a maximum capacity of 80% or less;
introduce the BIHW bag of the previous paragraph in a high-performance bag and seal hermetically by folding and banding;
irradiate the inside of the hermetically sealed metal container by means of the thermal conditioning means, controlling the temperature diffusion in coordinates;
to generate steam from the sterilizing solution;
(vii) bring the steam generated from the sterilizing solution into contact with the LLINs; and
viii) maintaining predetermined pressure and temperature conditions over the period of time t0 to t1.

In preferred embodiments, the thermal conditioning means of the sterilization and disinfection method are microwave emitters or are electrical resistors.

In a preferred embodiment of the method, t1 satisfies the ratio: 0.5 minutes ≤ t1 ≤ 2 minutes.

In another preferred embodiment of the method, the sterilizing solution is selected from the group comprising a) water, and b) aqueous solution of hydrogen peroxide and oxalic acid, wherein embodiment b) is preferably an aqueous solution containing 5 to 7% hydrogen peroxide, 4 to 6% oxalic acid and the remainder water.

For its part, the partially reflective gradient surface coating is obtained by performing at least one application of epoxy resin on the internal floor of the metal container, to which is associated an aluminum alloy grid as described above and achieve a partially reflective surface with a gradient phase behavior. For experimental validation, the highly reflective surfaces are used as reflectors inside the metal container. An improvement for thermal diffusion control is achieved by controlling the incidental wave distribution in a desired direction and direction by means of the plurality of baffles at the entrance of the container.

### EXAMPLES

Although microwaves are absorbed and deflected by the BIHW load, the system is configured to control temperature diffusion in coordinates by directing the incidence of waves directly through the BIHW load. This was tested by using a fixed volume of water at a known initial temperature and heating that sample for a known period of time and then measuring the temperature change in the volume of water once the sample cycle was completed, under standard pressure and time conditions for sterilization.

EXAMPLE 1. Loads of BIHW that meet the heterogeneity criterion, comprising sharps and disposable plastic utensils used to contain sharps, dry gauze and wet gauze, are placed in the bag and do not exceed 80% of the capacity of the BIHW bag. It is placed together with a container of sterilizing solution inside a hermetically sealed high-performance bag (4) and the metal container is closed.

Example 2. Temperature is measured by infrared sensors under the estimation of the embodiment wherein the plurality of diverters comprises only extended surfaces with highly reflective portions and: at least two of the extended surfaces comprise highly microwave reflective portions with parallel orientation and both with angular tilt within a range of 25° to 55°; at least one of the extended surfaces comprises highly microwave reflective portions with angular tilt greater than 90°, preferably within a range of 125° to 150°; at least one of the extended surfaces comprises downwardly facing highly microwave reflective portions with a horizontal arrangement at the top of the metal container; in this example, the first extended surface comprising a downwardly facing highly microwave reflective portion and said extended surface has an angular inclination of between 25° to 55°.

Example 3. The thermal conditioning means employed to generate the thermal energy in the system is by means of a microwave emitter. Since radio frequency heating requires the sample to be in the path of the microwave pattern, the position of the sample and the distribution of the microwaves within the container are important. A series of tests to determine optimum sterilization conditions is outlined below.

Example 4. A series of ten tests were performed, in between which the tightly sealed metal container and air were allowed to return to room temperature to eliminate the error caused by continuous heating. These tests focus on the configurations of the interior of the hermetically sealed metal container to increase microwave heating efficiency and are not intended to qualify the sterilization process, but rather to monitor the temperature diffusion in coordinates and to be able to determine its corresponding interaction with a point inside the container.

For each demonstration test, 500 mi of water was placed in a 1000 mi open top polypropylene container. The container was shaken and the temperature was measured for a period of 30 seconds or until the temperature measurement stabilized. The temperature was recorded and the container with water was placed in the container. The container was sealed and the RF thermal conditioning media was activated for a period of 120 seconds. Once the run time of said thermal conditioning means expired, the sample was quickly removed from the container and agitated with the temperature probe. The temperature was measured for a period of 30 seconds and recorded once the temperature reading stabilized. The temperature change was recorded and the average heat output was calculated as a function of run time, temperature change and water volume. Individual changes were then made to the geometry and placement of the plurality of diverters inside the hermetically sealed metal container and the test was repeated with a new water sample. The following results were obtained, recorded and compared.

Each subsection below describes the test parameters, container modifications and any notable observations during each test. The section numbers correspond to the test numbers mentioned above:

### Test 4.1

A basal test was performed on a metal container with the magnetron waveguide (2.3) arranged at the entrance of the container and in contact with a first extended surface (divertor) comprising a highly microwave reflecting portion facing downward and where said extended surface has an angular inclination of between 25° to 55°.

### Test 4.2

To the basal system, a radio frequency divertor with a convex surface approximately 2 inches in diameter perpendicular to the exit divertor was added to the waveguide with the intention of dispersing the incidence of radio frequency waves throughout the container.

### Test 4.3

The RF diverter with convex surface was moved with angular inclination greater than 90°, opposite the waveguide towards the container wall to adjust the point at which the waves were scattered.

### Test 4.4

The RF diverter with convex surface was moved towards the diverter outlet of the waveguide to delay the dispersion of the standing wave inside the waveguide. The divertor with convex surface experienced significant heating and caused the magnet used to hold the convex surface to the waveguide to break.

### Test 4.5

The convex surface diverter was removed and two of the extended surfaces comprising highly microwave reflective portions were installed in parallel orientation and both with angular inclination within a range of 25° to 55°, where the highly reflective portions face each other to reflect the waves through the container.

### Test 4.6

The diverter from Test 5 was retained and the convex surface diverter was reinstalled on the outlet at the Test 2 position.

### Test 4.7

Retained the diverter from test 5 and reinstalled the convex surface diverter under the waveguide outlet.

### Test 4.8

Added a second baffle plate.

### Test 4.9

Removed the convex surface baffle on the primary baffle and retained the second baffle.

### Test 4.10

Added flat baffle inside the container lid and retained the two baffles satisfying the definition of two extended surfaces comprising highly microwave reflective portions with parallel orientation and both with angular inclination within a range of 25° to 55°, where such highly reflective portions face each other to reflect waves through the container.

**Table 2.**

| # Test | Initial temp (°C) | Temp final (°C) | AT(-C) | Temperature Diffusion Control Microwave Performance (W) |
|---|---|---|---|---|
| 1 | 28.9 | 51.9 | 23 | 391.19 |
| 2 | 27.5 | 58.8 | 31.3 | 532.36 |
| 3 | 29.4 | 56.2 | 26.8 | 455.36 |
| 4 | 30.6 | 58 | 27.4 | 466.02 |
| 5 | 31.6 | 67.3 | 35.7 | 607.19 |
| 6 | 31.7 | 67.3 | 35.6 | 605.49 |
| 7 | 31.6 | 67.3 | 35.7 | 607.19 |
| 8 | 31.6 | 60.3 | 28.7 | 488.13 |
| 9 | 33.5 | 68.1 | 34.6 | 588.48 |
| 10 | 33.3 | 72.1 | 38.8 | 659.92 |

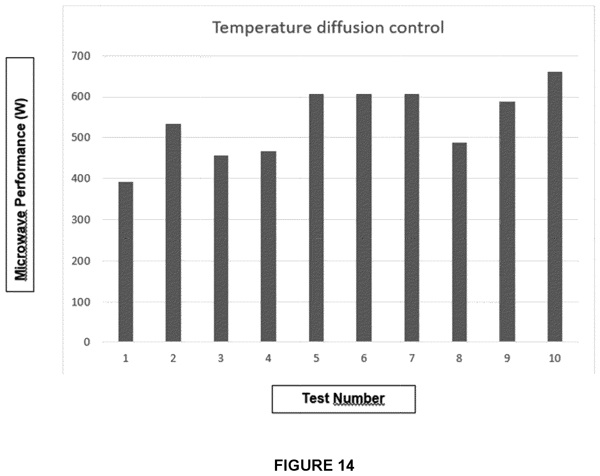

It was observed that the configuration of the container and internal baffle panels used in Test 10, shown in Figure 3, produces the highest temperature differential (TA) of any of the other configurations tested as shown in Figure 14 and thus exhibits directed coordinate temperature diffusion, whereas with modes 2 through 9, such diffusion exhibits dispersion behavior.

A part of the description of this application contains material subject to industrial property rights protection. The owner of such rights has no objection to the reproduction by facsimile of the patent document or of the description of the application by any person, as it appears in the patent file or records at the Patent and Trademark Office, but otherwise reserves all industrial property rights.

## Claims

1. - A mobile sterilization and disinfection system **characterized in that** it comprises:
- means for determining the mass of the load of BIHW at the place where they were generated;
a function control interface for interaction between the mobile system and the user;
- a hermetically sealed metal container comprising: a high-performance bag inside which there is a BIHW bag for a maximum load of 80% or less, a plastic container with sterilizing solution for steam generation and the BIHW;
- pressure control means;
- thermal conditioning means;
wherein:
the thermal conditioning means comprise:
a plurality of radio frequency diverters configured to control the temperature diffusion in coordinates;

2. - The mobile sterilization and disinfection system according to claim 1, **characterized in that** the plurality of diverters comprises extended surfaces arranged on at least one inner surface of the metal container, the extended surfaces being in contact with microwaves, and said extended surfaces are oriented to form a temperature gradient inside the metal container.

3. - The mobile sterilization and disinfection system according to claim 2, **characterized in that** the extended surfaces include highly microwave reflective portions, defined orientation and angular inclinations for coordinate temperature diffusion control.

4. - The mobile sterilization and disinfection system according to claim 3, **characterized in that** at least two of the extended surfaces comprise highly microwave reflective portions with parallel orientation and both, with angular inclination within a range of 25° to 55°, preferably of between 35° to 45°, and more preferably of between 40° to 45°, wherein said highly reflective portions face each other.

5. The mobile sterilization and disinfection system according to claim 3, **characterized in that** at least one of the extended surfaces comprises highly microwave reflective portions with angular inclination exceeding 90°, preferably within an interval from 125° to 150°, more preferably from 125° to 130°.

6. The mobile sterilization and disinfection system according to claim 3, **characterized in that** at least one of the extended surfaces comprises highly microwave reflective downwardly oriented portions with a horizontal arrangement arranged on the top of the metal container.

7. The mobile sterilization and disinfection system according to claim 1 **characterized in that** it additionally comprises cooling means, gas release means and filtration means.

8. The mobile sterilization and disinfection system according to claim 1 **characterized in that** the thermal conditioning means comprises at least one microwave emitter.

9. The mobile sterilization and disinfection system according to claim 1 **characterized in that** the thermal conditioning means are electrical heating elements.

10. The mobile sterilization and disinfection system according to claim 2 **characterized in that** the extended surfaces constituting the plurality of diverters are flat surfaces.

11. The mobile sterilization and disinfection system according to claim 2 **characterized in that** at least one of the extended surfaces constituting the plurality of diverters is a convex surface.

12. The mobile sterilization and disinfection system according to claim 1 **characterized in that** the plurality of radio frequency diverters additionally comprises a surface with metallic bumpers or humps partially reflecting electromagnetic waves.

13. The mobile sterilization and disinfection system according to claim 1 **characterized in that** the plurality of radio frequency diverters additionally comprises an electromagnetic wave partially reflective gradient surface coating defined by a grid composed with a first set of aluminum alloy strips in an arrangement oriented perpendicularly with respect to a second set of aluminum alloy strips, both sets being stamped on an epoxy resin substrate on the inner floor of the container.

14. The mobile sterilization and disinfection system according to claim 13 **characterized in that** the aluminum alloy comprises Cu 0.03-0.25%; Fe 0.2-0.5%; Mn 0.5-2. 0%; Si 0.05-0.5%, Zn 0.01-0.095% and unavoidable impurities 0.01-0.1% and balance of Al, preferably Cu 0.15%; Fe 0.4%; Mn 1.0%; Si 0.25%, Zn 0.05% and unavoidable impurities 0.05% and balance of Al.

15. The mobile sterilization and disinfection system according to claim 13 **characterized in that** the grid is printed on an epoxy resin substrate with a thickness of between 0.05mm and 0.4mm. The thickness of the aluminum strips has, in turn, a thickness of between 0.5mm and 0.4mm, preferably 0.2mm.

16. - The mobile sterilization and disinfection system according to claim 8 **characterized in that** the at least one microwave emitter magnetron (2") is located outside the metal container (3) , these two components, the metal container (3) and the at least one magnetron (2") being joined by a magnetron mounting waveguide with locking flange (2.2).

17. - The mobile sterilization and disinfection system according to claim 16 **characterized in that** the magnetron mounting waveguide (2.3) with locking flange (2.2) contains a microwave guide pressure window, wherein the window material is unfilled extruded polyetherimide (PEI).

18. - The mobile sterilization and disinfection system according to claim 17 **characterized in that** one end of the magnetron waveguide (2.3) is arranged at the entrance of the container and in contact with a first extended surface comprising a highly microwave reflecting portion facing downwards and wherein said extended surface has an angular inclination of between 25° to 55°, preferably of between 35° to 45°, and more preferably of between 40° to 45°.

19. - The mobile sterilization and disinfection system according to claim 1 **characterized in that** the sterilizing solution is selected from the group comprising a) water, b) aqueous solution of hydrogen peroxide and oxalic acid and c) saline solution.

20. - The mobile sterilization and disinfection system according to claim 19 **characterized in that** the aqueous hydrogen peroxide solution contains 5 to 7% hydrogen peroxide, 4 to 6% oxalic acid and water balance.

21. - The mobile sterilization and disinfection system according to claim 1 **characterized in that** it additionally comprises floor disinfection UV emitters.

22. - The mobile sterilization and disinfection system according to claim 1 **characterized in that** the high-performance bags are provided with an electronic traceability system selected from the group comprising QR codes, barcode, SD card, microwave resistant radio frequency identifier, (RFID), GPS locator or any other means of traceability.

23. The method of sterilization and disinfection according to the mobile sterilization system of claim 1 **characterized in that** comprising the steps of:
i) determine the mass of the BIHW load at the site where the waste was generated;
ii) calculate, by means of a control interface, the sterilization and disinfection conditions according to the requirements of step i);
iii) to introduce a load of BIHW and a plastic container with sterilizing solution in a BIHW bag at a maximum capacity of 80% or less;
iv) introduce the BIHW bag of the previous paragraph in a high-performance bag and seal hermetically by folding and banding;
v) irradiate the inside of the hermetically sealed metal container by means of the thermal conditioning means, controlling the temperature diffusion in coordinates;
vi) to generate steam from the sterilizing solution;
(vii) bring the steam generated from the sterilizing solution into contact with the BIHW; and
viii) to maintain predetermined pressure and temperature conditions in the period of time t0 a t1.

24. - The method of sterilization and disinfection according to claim 23 **characterized in that** it additionally comprises steps ix) cooling the interior of the metal container to room temperature and x) filtering the gases and vapors generated.

25. - The sterilization and disinfection method according to claim 23 **characterized in that** the thermal conditioning means are microwave emitters.

26. - The method of sterilization and disinfection according to claim 23 **characterized in that** the thermal conditioning means are electrical resistors.

27. - The method of sterilization and disinfection according to claim 23 **characterized in that** to = Start of cycle and ti satisfies the relationship: 0.5 minutes < t1 ≤ 2 minutes.

28. - The method of sterilization and disinfection according to claim 23 **characterized in that** the sterilizing solution is selected from the group comprising a) water, b) aqueous solution of hydrogen peroxide and oxalic acid and c) saline solution.

29. - The sterilizing and disinfecting method according to claim 23 **characterized in that** the aqueous hydrogen peroxide solution contains 5 to 7% hydrogen peroxide, 4 to 6% oxalic acid and water balance.
